(19) Europäisches Patentamt
European Patent Office
Office européen des brevets

(11) Veröffentlichungsnummer: 0 198 919
B1

(12) # EUROPÄISCHE PATENTSCHRIFT

(45) Veröffentlichungstag der Patentschrifft: 28.02.90

(51) Int. Cl.[5]: A 61 K 31/40, A 61 K 31/42, C 07 D 263/24

(21) Anmeldenummer: 85905773.9

(22) Anmeldetag: 18.10.85

(86) Internationale Anmeldenummer: PCT/DE 85/00472

(87) Internationale Veröffentlichungsnummer: WO 86/02268 (24.04.86 Gazette 86/09)

(54) ## PHARMAZEUTISCHE PRÄPARATE.

(30) Priorität: 19.10.84 DE 3438839

(43) Veröffentlichungstag der Anmeldung: 29.10.86 Patentblatt 86/44

(45) Bekanntmachung des Hinweises auf die Patenterteilung: 28.02.90 Patentblatt 90/09

(84) Bennante Vertragsstaaten: AT BE CH DE FR GB IT LI LU NL SE

(56) Entgegenhaltungen:
US-A-4 012 495
US-A-4 186 129

Journal of Heterocyclic Chemistry, Band 15, Nr. 3, Mai 1978, Provo, Utah, US, R.K. Jaiswal:"Substituted 5-aryl or 5-diphenylmethyl-3-alkyl-2-oxazolidones as anticonvulsants", Seiten 519-521

(73) Patentinhaber: SCHERING AKTIENGESELLSCHAFT Berlin und Bergkamen
Müllerstrasse 170/178 Postfach 65 03 11
D-1000 Berlin 65 (DE)

(72) Erfinder: KLOSE, Walter
Beatestrasse 9g
D-1000 Berlin 27 (DE)
Erfinder: KIRSCH, Gerald
Luciusstrasse 6B
D-1000 Berlin 33 (DE)
Erfinder: HUTH, Andreas
Kirchweg 55
D-1000 Berlin 38 (DE)
Erfinder: FRÖHLICH, Wolfgang
Westfälische Strasse 62
D-1000 Berlin 31 (DE)
Erfinder: LAURENT, Henry
Glambecker Weg 21
D-1000 Berlin 28 (DE)

EP 0 198 919 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBERGRAF, STOCKHOLM 1990

**Beschreibung**

Die Erfindung betrifft die Verwendung von Pyrrolidon und Oxazolidon-Derivaten der allgemeinen Formel I

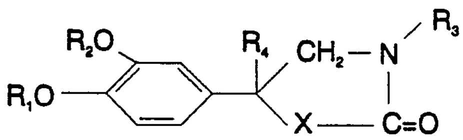

(I),

worin

X ein Sauerstoffatom oder eine Methylengruppe,
$R_1$ und $R_2$ ein Wasserstoffatom oder einen gegebenenfalls durch ein Sauerstoffatom unterbrochenen Kohlenwasserstoffrest mit maximal acht Kohlenstoffatomen,
$R_3$ ein Wasserstoffatom oder ein gegebenenfalls durch eine Oxogruppe substituierter Kohlenwasserstoffrest mit maximal acht Kohlenstoffatomen und
$R_4$ ein Wasserstoffatom oder eine Alkylgruppe mit maximal vier Kohlenstoffatomen bedeuten,

zur Herstellung eines Arzneimittels für die lokale Behandlung von Entzündungen.

Zahlreiche Verbindungen der allgemeinen Formel I gemäß Patentanspruch 1 sind vorbekannt. So werden beispielsweise in der US-A-4 012 495 Pyrrolidon-Derivate der allgemeinen Formel I und in der US-A-4 186 129 Oxazolidinon-Derivate der allgemeinen Formel I beschrieben. Diesen Patentschriften kann man entnehmen, daß sich diese Verbindungen durch eine zentral-depressive, antidopaminerge, antinoziceptive und antikonvulsive Wirksamkeit auszeichnen und darüberhinaus starke phosphodiesterasehemmende Eigenschaften haben.

Es wurde nun gefunden, daß die Verbindungen der allgemeinen Formel I gemäß Patentanspruch 1 bei topischer Applikation eine starke antiinflammatorische Wirksamkeit besitzen.

Antiinflammatorisch wirksame Verbindungen der allgemeinen Formel I gemäß Patentanspruch 1 sind solche, die als Substituenten $R_1$ und $R_2$ ein Wasserstoffatom oder einen gegebenenfalls durch ein Sauerstoffatom unterbrochenen Kohlenwasserstoffrest mit maximal acht Kohlenstoffatomen tragen. Die Substituenten $R_1$ und $R_2$ können gleich oder verschieden sein. Geeignete Kohlenwasserstoffreste $R_1$ und $R_2$ sind gesättigte oder ungesättigte, alicyclische, cycloaliphatische oder aromatische Kohlenwasserstoffreste, wie zum Beispiel die Methylgruppe, die Ethylgruppe, die Propylgruppe, die Isopropylgruppe, die Butylgruppe, die sek.-Butylgruppe, die Isobutylgruppe, die tert. Butylgruppe, die Pentylgruppe, die Hexylgruppe, die Allylgruppe, die 2-Propinylgruppe, die Cyclopentylgruppe, die Cyclohexylgruppe, die Cyclopropylmethylgruppe, die Cyclopentylmethylgruppe, die Cyclopentenylmethylgruppe, die Phenylgruppe oder die Benzylgruppe. Geeignete, durch ein Sauerstoffatom unterbrochene Kohlenwasserstoffreste $R_1$ und $R_2$ sind beispielsweise die 2-Tetrahydrofuranylgruppe und die 2-Tetrahydropyranylgruppe. Als Substituent $R_1$ eignet sich insbesondere der Methylrest. Als Kohlenwasserstoffreste $R_3$ können die Verbindungen der allgemeinen Formel I gemäß Patentanspruch 1 die gleichen Gruppen enthalten, die für die Substituenten $R_1$ und $R_2$ aufgeführt sind. Geeignete durch eine Oxogruppe substituierte Kohlenwasserstoffreste sind insbesondere in α-Position substituierte Kohlenwasserstoffreste, d. h. Acylgruppen. Geeignete Acylgruppen sind solche die sich von gesättigten oder ungesättigten, aliphatischen, cycloaliphatischen oder aromatischen Kohlenwasserstoffen mit maximal acht Kohlenstoffatomen ableiten, wie zum Beispiel 1-Oxoalkylgruppen (Formyl-, Acetyl-, Propionyl-, Butyryl-, Isobutyryl-, Trimethylacetylgruppen etc.), 1-Oxoalkyl-cycloalkylgruppen, wie die Cyclopentylcarbonylgruppe, die Cyclohexylcarbonylgruppe oder die 3-Cyclopropyl-1-oxo-propylgruppe, oder aromatische Acylgruppen, wie die Benzylgruppe oder 4-Methylbenzylgruppe.

Geeignete Alkylgruppen $R_4$ sind beispielsweise die Ethylgruppe, die Propylgruppe, die Isopropylgruppe oder insbesondere die Methylgruppe.

Zuvor unbekannte Verbindungen der allgemeinen Formel I gemäß Patentanspruch 1 sind die im Patentanspruch 4 gekennzeichneten Oxazolidinon-Derivate der allgemeinen Formel Ia. Diese Substanzen können in gleicher Weise hergestellt werden, wie die vorbekannten Oxazolidinon-Derivate, indem man ein Acetophenon der allgemeinen Formel II

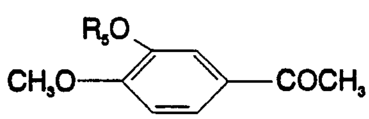

(II),

worin R die im Patentanspruch 4 genannte Bedeutung besitzt, in ein Cyanhydrin der allgemeinen Formel III

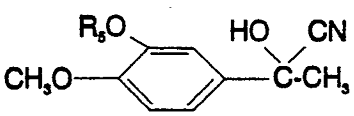

(III),

überführt, dies zum Amin der Formel IV

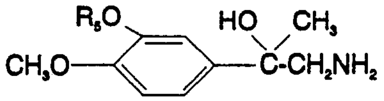

(IV),

reduziert und dieses mit einer Carbonylverbindung der allgemeinen Formel V

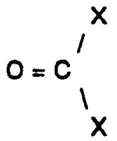

(V),

worin X jeweils Chloratome, niedere Alkoxygruppen (vorzugsweise Methoxygruppen oder Ethoxygruppen) oder 1-Imidazolylreste darstellen, kondensiert. Diese Reaktionsfolge kann beispielseise unter den Bedingungen durchgeführt werden, die im US-A-4 186 129 beschrieben sind.

Die antiinflammatorische Wirksamkeit der Verbindungen der allgemeinen Formel I gemäß Patentanspruch 1 wurde mittels des Rattenohr-Tests nach Tonelli (Endocrinol. 77, 1965, 625 und Proc. Soc. Exp. Med., 159, 1978, 223) wie folgt bestimmt:

Unterschiedliche Konzentrationen an Prüfsubstanz wurden in einer 5 Vol % ethanolische Crotonöl-Lösung gelöst oder durch fünfminutiges Behandeln im Ultraschallbad dispergiert. Pro Konzentration wurden jeweils 50 µl dieser Lösung und substanzfreier Crotonöl-Lösung mit einer 1 ml Tuberkulin-Spritze auf die beiden äußeren Ohrseiten von 10 narkotisierten Wistar-Ratten (Gewicht 160 bis 200 g) aufgetragen. Eine unbehandelte Gruppe diente als Kontrolle.

Fünf Stunden nach der Behandlung wurden die Tiere durch Behandeln mit $CO_2$-Gas getötet, die Ohren abgeschnitten und paarweise gewogen.

Als Maß für die topische entzündungshemmende Wirkung der Testsubstanzen wurde jeweils die prozentuale Hemmung der durch das Crotonöl hervorgerufenen Gewichtszunahme ermittelt.

Die nachfolgenden Tabellen zeigen die in diesem Test erhaltenen Ergebnisse.

**Tabelle 1**

| Nr. | Verbindung | $ED_{50}$[mg/kg] |
|---|---|---|
| 1 | Bufexamac | 15,0 |
| 2 | Bendazac | 15,0 |
| 3 | Hydrocortison-17-butyrat | 0,5 |
| 4 | 4-(3-Cyclopentyloxy-4-methoxyphenyl)-2-pyrrolidon | 0,6 |
| 5 | 4-[4-Methoxy-3-(3-tetrahydrofuryloxy)-phenyl]-2-pyrrolidon | 0,2 |
| 6 | 4-(3-Isobutoxy-4-methoxyphenyl)-2-pyrrolidon | 1,1 |
| 7 | 5-Methyl-5-[4-methoxy-3-(3-tetrahydro-pyranyloxy)-phenyl]-oxazolidinon | 0,18 |
| 8 | 5-(3-Cyclopropylmethoxy-4-methoxy-phenyl)-3,5-dimethyl-2-oxazolidinon | 0,22 |
| 9 | 5-(3-Ethoxy-4-methoxy-phenyl)-3,5-di-methyl-2-oxazolidinonen | 0,18 |
| 10 | 5-(3-Propinyloxy-4-methoxyphenyl)-2-oxazolidinon | 2,0 |
| 11 | 5-(3,4-Dimethoxyphenyl)-5-methyl-2-oxazolidinon | 0,6 |
| 12 | 5-(3-Cyclopentyloxy-4-methoxypenyl)-2-oxazolidinon | 1,0 |
| 13 | 5-(3-Cyclopentyloxy-4-methoxyphenyl)-5-methyl-2-oxazolidinon | 1,2 |
| 14 | 5-[3-(3-Tetrahydrofuryloxy)-4-methoxy-phenyl]-2-oxazolidinon | 0,5 |
| 15 | 5-(4-Methoxy-3-propoxyphenyl)-2-oxazolidinon | 1,3 |
| 16 | 5-(3-Allyloxy-4-methoxyphenyl)-2-oxazolidion | 3,2 |
| 17 | 5-Methyl-5-(3-cyclopropylmethoxy-4-methoxyphenyl)-2-oxazolidinon | 0,5 |
| 18 | 5-Methyl-5-(4-methoxy-3-propoxyphenyl)-2-oxazolidinon | 2,3 |
| 19 | 5-Methyl-5-(3-butoxy-4-methoxyphenyl)-2-oxazolidinon | 2,7 |
| 20 | 5-Methyl-5-(3-isopropoxy-4-methoxyphenyl-2-oxazolidinon | 1,7 |
| 21 | 5-Methyl-1-5-(3-ethoxy-4-methoxyphenyl-2-oxazolidinon | 1,3 |

Die aufgeführten Ergebnisse zeigen, daß die Verbindungen der allgemeinen Formel I bei topischer Applikation wesentlich stärker antiinflammatorisch wirksam sind als die vorbekannten vergleichbaren Substanzen 1 und 2. Sie erreichen fast die Wirkungsintensität von stark wirksamen Kortikoiden wie etwa Hydrocortison-17-butyrat (Substanz 3).

Die Herstellung der Arzneimittelspezialitäten erfolgt in üblicher Weise, indem man die Wirkstoffe mit geeigneten Zusätzen in die gewünschte Applikationsform, wie zum Beispiel: Lösungen, Lotionen, Salben, Cremes oder Pflaster, überführt. In den so formulierten Arzneimitteln ist die Wirkstoffkonzentration von der Applikationsform abhängig. Bei Lotionen Cremes und Salben wird vorzugsweise eine Wirkstoffkonzentration von 0,01 % bis 10 % verwendet.

Die Lotionen oder Cremes (Öl/Wasser-Emulsionen) und die Salben (Wasser/Öl-Emulsionen) können in konventioneller Weise unter Verwendung konventioneller Emulgatoren hergestellt werden (Kirk Othmer: Enzyclopedia of Chemical Technology, 3. Auflage, 1979; John Wiley & Sons; New York etc. Vol 8, Seite 900 bis 930 und Dr. Otto-Albrecht Neumüller: Römpps Chemie Lexikon 7. Auflage, 1973; Franck'sche Verlagshandlung Stuttgart, Seite 1009 bis 1013). Die für diese Emulsionen verwendeten Wachse, Emulgatoren und übrigen Zusätze sind die gleichen, wie man sie konventioneller Weise verwendet (Dr. Otto-Albrecht Neumüller: Römpps Chemie Lexikon, 7. Auflage 1973; Franck'sche Verlagshandlung Stuttgart, Seite 1427 und 1428).

Ein erfindungsgemäßes pharmazeutisches Präparat in Form einer Öl/Wasser-Emulsion kann aus hydrophilen und/oder lipophilen Wirkstoffen, Fettphase, Öl/Wasser-Emulgator, wässriger Phase und Konservierungsmittel bestehen.

Als hydrophile und/oder lipophile Wirkstoffe können Feuchthaltefaktoren (Hydrokomplexe), wie zum Beispiel Glycerin, Polyäthylenglykole oder Aminosäuregemische, Puroba-Oil (= Jojoba-Öl), Vitamine (vorzugsweise Vitamin A und E) Vitalkomplexe (wie zum Beispiel Placentaextrakte), Enzyme, Kräuterauszüge (wie zum Beispiel Hammamelis Extrakt oder Kamillenextrakt) oder Proteine (wie zum Beispiel Collagen) eingesetzt werden. Als ölige Phase oder als Fettphase in der Öl/Wasser-Emulsion eigen sich Kohlenwasserstoffe wie zum Beispiel Vaseline, Paraffine oder Stearin, oder Wachse wie zum Beispiel Bienenwachs. Geeignete Öl/Wasser-Emulgatoren sind beispielsweise Stearylalkohol, Polyoxyäthylenstearate (wie zum Beispiel MYRJ(R)), Komplexemulgatoren (wie zum Beispiel Amphoterin(R)) und Sorbitanfettsäureester (wie zum Beispiel Span(R)) oder Carboxyvinylpolymerisate (wie zum Beispiel Carbopol(R)). Die wässrige Phase kann zusätzlich noch Puffersubstanzen, wie zum Beispiel das Dinatriumsalz der Äthylendiamin-N,N,N'-tetraessigsäure und Konservierungsmittel wie Chlorquinaldol, Parabene oder Benzalkoniumchlorid enthalten.

In der Öl/Wasser-Emulsion beträgt der Anteil der inneren Emulsion vorzugsweise 10 bis 49 Gew.%, die Teilchengröße der inneren Emulsion liegt vorzugsweise zwischen 1 µm und 100 µm.

Ein erfindungsgemäßes pharmazeutisches Präparat in Form einer Wasser/Öl-Emulsion besteht ebenfalls aus hydrophilen und/oder lipophilen Wirkstoffen, wie sie auch in der Öl/WasserEmulsion verwendet werden, Fettphase, Wasser/Öl-Emulgator und wässriger Phase. Als ölige Phase oder Fettphase der Wasser/Öl-Emulsion können Kohlenwasserstoffe, z. B. Paraffine und Vaseline, synthetische, pflanzliche und tierische Öle bzw. Wachse (wie zum Beispiel Olivenöl, Erdnußöl, feines Knochenöl, Mandelöl, Lanolin, Bienenwachs oder Sonnenblumenöl) eingesetzt werden, als wässrige Phase gereinigtes demineralisiertes Wasser und als Wasser/Öl-Emulgator Wollfett (=Lanolin), Fettalkohole wie zum Beispiel Cetylalkohol, Myristylalkohol, Stearylalkohol oder Cerylalkohol, Fettsäureester, wie zum Beispiel Bienenwachs (Cera alba) oder Wachsalkoholester oder Mischester (wie zum Beispiel Dehymuls(R).

In der Wasser/Öl-Emulsion beträgt der Anteil der inneren Emulsion vorzugsweise 30 bis 49 Gew. %, die Teilchengröße der inneren Emulsion liegt vorzugsweise zwischen 1 µm und 100 µm.

Das feindisperse System wird zusätzlich mit dem mikronisierten Wirkstoff (Korngröße vorzugsweise 1 bis 20 µm) und gegebenenfalls noch mit Duftstoffen, wie zum Beispiel diejenige der Crematest(R)-Serie versetzt und bis zur gleichmäßigen Verteilung derselben gerührt.

Darüberhinaus sind die Verbindungen der allgemeinen Formel I gemäß Patentanspruch 1 gegebenenfalls in Kombination mit den üblichen Trägermitteln und Hilfsstoffen auch zur Herstellung von Inhalationsmitteln geeignet, welche zur Therapie allergischer Erkrankungen der Atemwege wie zum Beispiel des Bronchialasthmas oder der Rhinitis verwendet werden können.

Ferner eignen sich die Verbindungen der Formel I auch in Form von Kapseln, Tabletten oder Dragees, die vorzugsweise 10 bis 200 mg Wirkstoff enthalten und oral appliziert werden oder in Form von Suspensionen, die vorzugsweise 100 bis 1500 mg Wirkstoff pro Dosiseinheit enthalten und rektal appliziert werden auch zur Behandlung allergischer Erkrankungen des Darmtraktes, wie der Kolitis ulcerosa und der Kolitis granulomatosa.

Die nachfolgenden Ausführungsbeispiele dienen zur Erläuterung der Erfindung.

**1. Ausführungsbeispiele betreffend pharmazeutische Präparate**

**Beispiel 1**

a) Herstellung der Öl/Wasser-Emulsion.
10,00 g Dinatriumedetat und 10,00 g Chlorquinaldol werden in 300,00 g gereinigtem demineralisiertem Wasser gelöst und mit 10,00 g Carbopol(R) versetzt.
Diese Mischung wird unter kräftigem Rühren in eine Schmelze von 80,00 g Vaseline (DAB 8) – DAB ist die Abkürzung für das Deutsche Arzneibuch, amtliche Ausgabe, 8. Auflage 1978 – 40,00 g Stearylalkohol, 30,00 g MYRJ(R)

und 50,00 g Pur-oba-Öl eingetragen. Man rührt die Mischung noch solange, bis eine Emulsion mit einer Teilchengröße von 20 - 70 µm entsteht.

b) Herstellung der Wasser/Öl-Emulsion.
230,00 g gereinigtes demineralisiertes Wasser werden unter kräftigem Rühren in eine Schmelze von 220,00 g Vaseline (DAB 8), 10,00 g Dehymuls(R) und 10,00 g Cera alba eingetragen. Man rührt die Mischung noch solange, bis eine Emulsion mit einer Teilchengröße von 20 - 70 µm entsteht.

c) Herstellung einer Creme. Die Wasser/Öl-Emulsion wird unter kräftigem Rühren unter einem Vakuum von 10 torr in die Öl/Wasser-Emulsion eingetragen. Man rührt noch solange, bis eine Dispersion mit einer Teilchengröße von 10 bis 50 µm entsteht, und entfernt das Vakuum.
In 95,000 g dieser Salbengrundlage setzt unter Rühren 5,000 g 5-(3,4-Dimethoxyphenyl)-5-methyl-3-oxazolidinon – mikronisiert; Teilchengröße vorwiegend 1 bis 20 µm – zu und rührt bis sich der Wirkstoff in der Salbengrundlage gleichmäßig verteilt hat.

**Beispiel 2**

97,000 g der gemäß Beispiel 1c hergestellten Salbengrundlage wird mit 3,000 g 4-(3-Cyclopentyloxy-4-methoxy-phenyl)-2-pyrrolidon – mikronisiert; Teilchengröße vorwiegend 1 bis 20 µm – versetzt und gerührt bis sich der Wirkstoff in der Salbengrundlage gleichmäßig verteilt.

**Beispiel 3**

95,000 g der gemäß Beispiel 1c hergestellten Salbengrundlage wird mit 5,000 g 4-(3-Isobutoxy-4-methoxy-phenyl)-2-pyrrolidon – mikronisiert; Teilchengröße vorwiegend 1 bis 20 µm – versetzt und gerührt bis sich der Wirkstoff in der Salbengrundlage gleichmäßig verteilt.

**Beispiel 4**

97,500 g der gemäß Beispiel 1c hergestellten Salbengrundlage wird mit 2,500 g 5-[4-Methoxy-3-(2-tetrahydrofuranyloxy)-phenyl]-2-oxazolidinon – mikronisiert; Teilchengröße vorwiegend 1 bis 20 µm – versetzt und gerührt bis sich der Wirkstoff in der Salbengrundlage gleichmäßig verteilt.

**Beispiel 5**

95,000 g der gemäß Beispiel 1c hergestellten Salbengrundlage werden mit 5,000 g 5-Methyl-5(3-cyclopropylmethoxy-4-methoxy-phenyl)-2-oxazolidinon – mikronisiert; Teilchengröße vorwiegend 1 bis 20 µm – versetzt und gerührt bis sich der Wirkstoff in der Salbengrundlage gleichmäßig verteilt.

**Beispiel 6**

45,000 g Vaseline (DAB-8), 19,600 g Paraffinöl, 5,000 g Cetylalkohol, 5,000 g Bienenwachs und 5,000 g Sorbitansesquinolat werden zusammengeschmolzen, mit einer Lösung von 0,2000 g p-Hydroxybenzoesäureester in 15,2 g entmineralisiertem Wasser versetzt und bei 50°C emulgiert. Dann läßt man die Emulsion erkalten versetzt sie mit 5,000 g 4-(3-Cyclopentyloxy-4-methoxy-phenyl)-2-pyrrolidon – mikronisiert; Teilchengröße vorwiegend 1 bis 20 µm – und rührt bis sich der Wirkstoff in der Salbengrundlage gleichmäßig verteilt hat.

**Beispiel 7**

1,000 g mikronisiertes 4-(3-Cyclopentyloxy-4-methoxy-phenyl)-2-pyrrolidon (mittlere Korngröße: kleiner als 8 µm) und 39,000 g vermahlene Lactose werden gemischt. Zur Applikation des Inhalationsmittels verwendet man jeweils 200 mg des Gemisches.

**Beispiel 8**

Eine Sprühdose, welche mit einem Dosierventil (eine Dosis = 200 mg; dies entspricht 5 mg Wirkstoff) versehen ist, wird mit 250 mg 4-(3-Cyclopentyloxy-4-methoxy-phenyl)-2-pyrrolidon und 5,0 ml Frigen(R) 12/114 (40 : 60) gefüllt.

**Beispiel 9**

5,00 g Polyoxyethylenpolyoxypropylenpolymeres und 25,00 g Polyvinylpyrrolidon werden in 800 ml bidestilliertem Wasser gelöst, die Lösung mit 50,000 g 4-(3-Cyclopentyloxy-4-methoxy-phenyl)-2-pyrrolidon versetzt und homogenisiert. Dann füllt man die erhaltene Suspension mit bidestilliertem Wasser auf 2000 ml auf, gibt je 50 ml der Suspension in ein Infusionsfläschchen, friert die Proben 150 Minuten bei - 30°C ein und gefriergetrocknet sie 48 Stunden lang.

Vor Gebrauch wir die Probe in 50 ml bidestilliertem Wasser resuspendiert und diese Suspension rektal appliziert.

**2. Ausführungsbeispiele betreffend die Synthese der Oxa-zolidinon-Derivate der allgemeinen Formel Ia**

**Beispiel 1**

3,10 g (15,0 mMol) 3-Allyloxy-4-methoxyacetophenon, 2,18 ml (16,4 mMol) Trimethylsilylcyanid und 126 mg (0,39 mMol) Zinkjodid wurden 5 Stunden bei 100°C und 14 Stunden bei Raumtemperatur unter Argon gerührt. Die Reaktionsmischung wurde mit 4,6 ml Ether versetzt und zu 0,69 g (18,5 mMol) Lithiumaluminiumhydrid in 13,5 ml Ether getropft. Nach einer Rührzeit von 1 Stunde bei 40°C Badtemperatur wurde die Reaktionsmischung nacheinander mit 0,7 ml Wasser, 0,75 ml 4 N Natronlauge und mit 2,1 ml Wasser zersetzt. Der sich bildende Feststoff wurde mehrmals mit Ether ausgewaschen, die vereinigten Etherphasen über Natriumsulfat getrocknet, filtriert und eingeengt (3,33 g Öl). Nach erneuter Zugabe von 42 mg Zinkjodid und 0,73 ml Trimethylsilylcyanid wurden weitere 4 Stunden bei 100°C gerührt. Die Reaktionsmischung wurde mit 3 ml Ether verdünnt und zu einer Suspension aus 240 mg Lithiumaluminiumhydrid in 6 ml Ether getropft. Anschließend wurde bei 40°C 30 Minuten gerührt und danach die Reaktionsmischung mit 0,2 ml Wasser, 0,26 ml 4 N Natronlauge und 0,8 ml Wasser zersetzt. Der sich bildende Feststoff wurde mehrmals mit Ether gewaschen, die vereinigten Etherphasen über Natriumsulfat getrocknet, filtriert und eingeengt. Der ölige Rückstand (2,06 g) wurde in 31 ml Tetrahydrofuran mit 2,11 g (12,4 mMol) Carbonyldiimidazol 20 Stunden bei Raumtemperatur gerührt. Die Reaktionsmischung wurde im Vakuum eingeengt, der Rückstand in Essigester gelöst, die organische Lösung mit 2 N Salzsäure und Wasser gewaschen, über Natriumsulfat getrocknet, filtriert und eingeengt (1,52 g Öl). Durch Chromatographie an Kieselgel mit Methylenchlorid/Ether (1 : 1) wurden 260 mg 5-Methyl-5-(3-allyloxy-4-methoxyphenyl)-2-oxazolidinon als Öl erhalten.

$^1$H-NMR ($CDCl_3$): δ = 1,75 (s, 2H), 3,63 (s, 2H), 3,83 (s, 3H), 4,55 (pseudo d, 2H), 5,10 - 6,23 (m, 4H), 6,80 (pseudo d, 3H).

**Beispiel 2**

2,84 g (12,9 mMol) 3-Cyclopropylmethoxy-4-methoxyacetophenon, 1,87 ml (14,0 mMol) Trimethylsilylcyanid und 109 mg (0,33 mMol) Zinkjodid wurden 5 Stunden bei 100°C und 14 Stunden bei Raumtemperatur gerührt. Nach Zugabe von 4,5 ml Ether wurde zu 0,6 g (15,8 mMol) Lithiumaluminiumhydrid in 12 ml Ether getropft und 1 Stunde bei 40°C (Badtemperatur) gerührt. Die Reaktionsmischung wurde nacheinander mit 0,7 ml Wasser, 0,75 ml 4 N Natronlauge und 2,1 ml Wasser versetzt. Der sich bildende Feststoff wurde mehrmals mit Ether gewaschen, die vereinigten Etherphasen mit Natriumsulfat getrocknet, filtriert und im Vakuum eingeengt. Der Rückstand (2,31 g Öl) wurde mit 2,21 g (13,0 mMol) Carbonyldiimidazol in 33 ml Tetrahydrofuran 4 Stunden bei Raumtemperatur gerührt. Die weitere Durchführung erfolgte analog Beispiel 1. Es wurden 558 mg 5-Methyl-5-(3-cyclopropylmethoxy-4-methoxyphenyl)-2-oxazolidinon vom Schmelzpunkt 96 bis 97°C erhalten.

**Beispiel 3**

1,63 g (7,8 mMol) 3-Propoxy-4-methoxyacetophenon, 1,1 ml (8,5 mMol) Trimethylsilylcyanid und 66 mg (0,21 mMol) Zinkjodid wurden 4,5 Stunden bei 100°C und 14 Stunden bei Raumtemperatur gerührt. Die Reaktionsmischung wurde mit 4,6 ml Ether versetzt und zu 0,36 g (9,6 mMol) Lithiumaluminiumhydrid in 7 ml Ether getropft. Nach 1 Stunde Rührzeit bei 40° C (Badtemperatur) wurde die Reaktionsmischung nacheinander mit 0,4 ml Wasser, 0,4 ml 4 N Natronlauge und 1 ml Wasser zersetzt. Die weitere Durchführung analog dem Beispiel 1, nur wurden bei erneuter Zugabe der Reagenzien 20 mg Zinkjodid und 0,36 ml Trimethylsilylcyanid verwendet bzw. mit 0,1 ml Wasser, 0,13 ml 4 N Natronlauge und 0,4 ml Wasser zersetzt. Zur Carbonylierung wurden 1,19 g (7,0 mMol) Carbonyldiimidazol in 17,5 ml Tetrahydrofuran verwendet. Man erhielt 152 mg 5-Methyl-5-(4-methoxy-3-propoxyphenyl)-2-oxazolidinon als kristallines Öl. Umkristallisation in Essigester ergab einen Schmelzpunkt von 78°C.

**Beispiel 4**

3,47 g (15,6 mMol) 3-Butoxy-4-methoxyacetophenon, 2,26 ml (17,0 mMol) Trimethylsilylcyanid und 131 mg (0,91 mMol) Zinkjodid wurden 5 Stunden bei 100°C und 14 Stunden bei Raumtemperatur gerührt. Nach Zugabe von 4,6 ml Ether

wurde zu 0,73 g (19,2 mMol) Lithiumaluminiumhydrid in 14 ml Ether getropft und 1 Stunde bei 40°C (Badtemperatur) gerührt. Die weitere Durchführung erfolgte analog dem Beispiel 2, nur wurden 2,18 g (12,8 mMol) Carbonyldiimidazol in 32,5 ml Tetrahydrofuran verwendet. Es wurden 569 mg 5-Methyl-5-(3-butoxy-4-methoxyphenyl)-2-oxazolidinon vom Schmelzpunkt 108 - 109°C (aus Essigester) erhalten.

**Beispiel 5**

3,01 g (14,5 mMol) 3-Isopropyl-4-methoxyacetophenon, 2,1 ml (15,8 mMol) Trimethylsilylcyanid und 122 mg (0,38 mMol) Zinkjodid wurden 5 Stunden bei 100°C und 14 Stunden bei Raumtemperatur unter Argon gerührt. Die weitere Durchführung erfolgte analog dem Beispiel 1. Es wurden 456 mg 5-Methyl-5-(3-isopropoxy-4-methoxyphenyl)-2-oxazolidinon vom Schmelzpunkt 67 bis 69,5°C erhalten.

**Beispiel 6**

1,66 g (8,55 mMol) 3-ethoxy-4-methoxyacetophenon wurden mit 1,24 ml (9,3 mMol) Trimethylsilylcyanid und 72 mg (0,22 mMol) Zinkjodid 5 Stunden bei 100°C und 14 Stunden bei Raumtemperatur unter Argon gerührt. Die Reaktionsmischung wurde mit 2,5 ml Ether verdünnt und zu 0,4 g (10,5 mMol) Lithiumaluminiumhydrid in 8 ml Ether getropft. Nach einer Rührzeit von 1 Stunde bei 40°C (Badtemperatur) wurde die Reaktionsmischung nacheinander mit 0,45 ml Wasser, 0,45 ml 4 N Natronlauge und 1,1 ml Wasser zersetzt. Der sich bildende Feststoff wurde mehrmals mit Ether ausgewaschen und die vereinigten Etherphasen über Natriumsulfat getrocknet, filtriert und im Vakuum eingeengt. Der Rückstand (1,5 g) wurde in 24 ml Tetrahydrofuran mit 1,61 g (9,5 mMol) Carbonyldiimidazol 4 Stunden bei Raumtemperatur gerührt. Die Reaktionslösung wurde eingeengt, der Rückstand in Essigester gelöst, die organische Lösung mit 2 N Salzsäure und Wasser gewaschen, über Natriumsulfat getrocknet, filtriert und eingeengt (0,8 g Öl). Chromatographie an Kieselgel mit Methylenchlorid/Ether (1 : 1) ergab 370 mg 5-Methyl-5-(3-ethoxy-4-methoxyphenyl)-2-oxazolidinon vom Schmelzpunkt 63 bis 65°C.

**Beispiel 7**

3,7 g (15,7 mMol) 4-Methoxy-3-(3-tetrahydrofuryloxy-acetophenon, 2,3 ml (17,1 mMol) Trimethylsilyl-cyanid und 134 mg (0,40 mMol) Zinkjodid wurden 5 Stunden bei 100°C und 14 Stunden bei Raumtemperatur unter Argon gerührt. Die weitere Durchführung erfolgte analog dem Beispiel 1. Es wurden 600 mg 5-Methyl-5-[4-methoxy-3-(3-tetrahydrofuryloxy)-phenyl]-2-oxazolidinon als farbloses Öl erhalten.

**Beispiel 8**

500 mg (1,8 mMol) 5-Methyl-5-(3-cyclopropylmethoxy-4-methoxyphenyl)-2-oxazolidinon, 113 mg (2,0 mMol) Kaliumhydroxid und 0,15 ml (2,2 mMol) Methyljodid wurden in 4 ml Tetrahydrofuran 4 Stunden bei Raumtemperatur gerührt. Die Reaktionsmischung wurde filtriert, das Filtrat mit 30 ml Wasser versetzt und danach mit Essigester extrahiert. Die Essigester-Phase wurde mit Wasser gewaschen, über Natriumsulfat getrocknet, filtriert und eingeengt (390 mg gelbes Öl). Durch PSC-Trennung mit Methylenchlorid/Ether (1 : 1) wurden 212 mg 5-(3-Cyclo-propylmethoxy-4-methoxyphenyl)-3,5-dimethyl-2-oxazolidinon als Öl erhalten.

**Beispiel 9**

500 mg (2,0 mMol) 5-Methyl-5-(3-ethoxy-4-methoxyphenyl)-2-oxazolidinon, 125 mg (2,22 mMol) Kaliumhydroxid und 0,167 ml (2,38 mMol) Methyljodid wurden in 4 ml Tetrahydrofuran 4 Stunden bei Raumtemperatur gerührt. Die weitere Durchführung erfolgte analog dem Beispiel 8. Es wurden 201 mg 5-(3-Ethoxy-4-methoxyphenyl)-3,5-dimethyl-2-oxazolidinon als Öl erhalten.

**Beispiel 10**

500 mg (2,0 mMol) 5-Methyl-5-(3-ethoxy-4-methoxyphenyl)-2-oxazolidinon wurden zunächst mit 60 mg (2,5 mMol) Natriumhydrid in 14 ml Dimethylformamid 30 Minuten und nach Zugabe von 0,29 ml (3,0 mMol) (Brommethyl)-cyclopropan 17 Stunden bei Raumtemperatur gerührt. Die Reaktionsmischung wurde mit 50 ml Wasser versetzt und danach mit Essigester extrahiert. Die Essigester-Phase wurde mit Wasser gewaschen, über Natriumsulfat getrocknet, filtriert und eingeengt (766 mg gelbes Öl). Durch Chromatographie an Kieselgel mit Methylenchlorid/Ether (1 : 1) wurden 438 mg 5-Methyl-5-(3-ethoxy-4-methoxyphenyl)-3-cyclopropylmethyl-2-oxazolidinon als Öl erhalten.

**Beispiel 11**

500 mg (1,8 mMol) 5-Methyl-5-(3-cyclopropylmethoxy-4-methoxyphenyl)-2-oxazolidinon wurden zunächst mit 75 mg (3,1 mMol) Natriumhydrid in 17 ml Dimethylformamid 30 Minuten und nach Zugabe von 0,25 ml (2,7 mMol) Isopropylbromid 17 Stunden bei Raumtemperatur gerührt. Die weitere Durchführung erfolgte analog Beispiel 1. Es wurden 169 mg 5-Methyl-5-(3-cyclopropylmethoxy-4-methoxyphenyl)-3-isopropyl-2-oxazolidinon als Öl erhalten.

**Beispiel 12**

500 mg (1,8 mMol) 5-Methyl-5-(3-cyclopropylmethoxy-4-methoxyphenyl)-2-oxazolidinon wurden zunächst mit 75 mg (3,1 mMol) Natriumhydrid in 17 ml Dimethylformamid 30 Minuten und nach Zugabe von 0,32 ml (2,7 mMol) Benzylbromid 2 Stunden bei Raumtemperatur gerührt. Die weitere Durchführung erfolgte analog Beispiel 10. Es wurden 348 mg 5-Methyl-5-(3-cyclopropylmethoxy-4-methoxyphenyl)-3-benzyl-2-oxazolidinon als Öl erhalten.

**Patentansprüche**

1. Verwendung von Pyrrolidon- und Oxazolidon Derivaten der allgemeinen Formel I

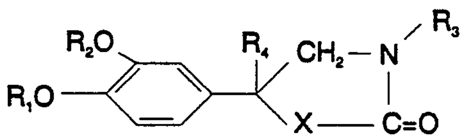

(I),

worin

X ein Sauerstoffatom oder eine Methylengruppe,
$R_1$ und $R_2$ ein Wasserstoffatom oder einen gegebenenfalls durch ein Sauerstoffatom unterbrochenen Kohlenwasserstoffrest mit maximal acht Kohlenstoffatomen,
$R_3$ ein Wasserstoffatom oder ein gegebenenfalls durch eine Oxogruppe substituierter Kohlenwasserstoffrest mit maximal acht Kohlenstoffatomen und
$R_4$ ein Wasserstoffatom oder eine Alkylgruppe mit maximal vier Kohlenstoffatomen bedeuten,
zur Herstellung eines Arzneimittels für die lokale Behandlung von Entzündungen.

2. Verwendung von Pyrrolidon- und Oxazolidon-Derivaten der allgemeinen Formel I gemäß Patentanspruch 1 zur Herstellung eines Arzneimittels für die lokale Behandlung von Entzündungen in Form einer Lösung, Salbe, Creme oder Lotion.

3. Verwendung von Pyrrolidon- und Oxazolidon-Derivaten der allgemeinen Formel I gemäß Patentanspruch 1 zur Herstellung eines Arzneimittels für die lokale Behandlung von Entzündungen in Form eines Pulvers.

4. Oxazolidinon-Derivate der allgemeinen Formel Ia

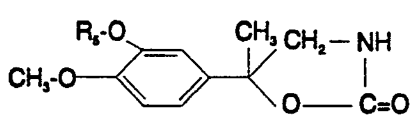

(Ia),

worin $R_5$ ein Kohlenwasserstoffrest mit zwei bis vier Kohlenstoffatomen bedeutet.

5. 5-Methyl-5-(3-allyloxy-4-methoxyphenyl)-2-oxazolidinon.

6. 5-Methyl-5-(3-cyclopropylmethoxy-4-methoxyphenyl)-2-oxazolidinon.

7. 5-Methyl-5-(4-methoxy-3-propoxyphenyl)-2-oxazolidinon.

8. 5-Methyl-5-(3-butoxy-4-methoxyphenyl)-2-oxazolidinon.

9. 5-Methyl-5-(3-isopropoxy-4-methoxyphenyl)-2-oxazolidinon.

10. 5-Methyl-5-(3-ethoxy-4-methoxyphenyl)-2-oxazolidinon.

11. 5-Methyl-5-[-4-methoxy-3-(3-tetrahydrofuranyloxy)-phenyl]-oxazolidinon.

12. 5-(3-Cyclopropylmethoxy-4-methoxyphenyl)-3,5-dimethyl-2-oxazolidinon.

13. 5-(3-Ethoxy-4-methoxyphenyl)-3,5-dimethyl-2-oxazolidinon.

14. 5-Methyl-5-(3-ethoxy-4-methoxyphenyl)-3-cyclopropylmethyl-2-oxazolidinon.

15. 5-Methyl-5-(3-cyclopropylmethoxy-4-methoxyphenyl)-3-isopropyl-2-oxazolidinon.

16. 5-Methyl-5-(3-cyclopropylmethoxy-4-methoxyphenyl)-3-benzyl-2-oxazolidinon.

17. Verwendung von Oxazolidon Derivaten gemäß Patentanspruch 4 bis 16 zur Herstellung eines Arzneimittels für die lokale Behandlung von Entzündungen.

**Claims**

1. The use of pyrrolidone and oxazolidone derivatives of the general formula I

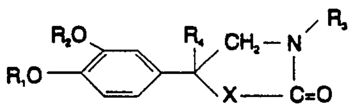

(I),

in which

X represents an oxygen atom or a methylene group,
$R_1$ and $R_2$ represent a hydrogen atom or a hydrocarbon radical having a maximum of eight carbon atoms that is optionally interrupted by an oxygen atom,
$R_3$ represents a hydrogen atom or a hydrocarbon radical having a maximum of eight carbon atoms that is optionally substituted by an oxo group, and
$R_4$ represents a hydrogen atom or an alkyl group having a maximum of four carbon atoms,
for the preparation of a medicament for the local treatment of inflammations.

2. The use of pyrrolidone and oxazolidone derivatives of the general formula I according to patent claim 1 for the preparation of a medicament for the local treatment of inflammations in the form of a solution, ointment, cream or lotion.

3. The use of pyrrolidone and oxazolidone derivatives of the general formula I according to patent claim 1 for the preparation of a medicament for the local treatment of inflammations in the form of a powder.

4. Oxazolidinone derivatives of the general formula Ia

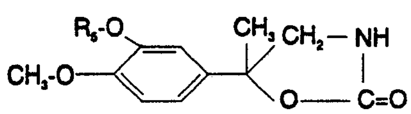

(Ia),

in which $R_5$ is a hydrocarbon radical having from two to four carbon atoms.

5. 5-Methyl-5-(3-allyloxy-4-methoxyphenyl)-2-oxazolidinone.

6. 5-Methyl-5-(3-cyclopropylmethoxy-4-methoxyphenyl)-2-oxazolidinone.

7. 5-Methyl-5-(4-methoxy-3-propoxyphenyl)-2-oxazolidinone.

8. 5-Methyl-5-(3-butoxy-4-methoxyphenyl)-2-oxazolidinone.

9. 5-Methyl-5-(3-isopropoxy-4-methoxyphenyl)-2-oxazolidinone.

10. 5-Methyl-5-(3-ethoxy-4-methoxyphenyl)-2-oxazolidinone.

11. 5-Methyl-5-[4-methoxy-3-(3-tetrahydrofuranyloxy)-phenyl]-oxazolidinone.

12. 5-(3-Cyclopropylmethoxy-4-methoxyphenyl)-3, 5-dimethyl-2-oxazolidinone.

13. 5-(3-Ethoxy-4-methoxyphenyl)-3, 5-dimethyl-2-oxazolidinone.

14. 5-Methyl-5-(3-ethoxy-4-methoxyphenyl)-3-cyclopropyl-methyl-2-oxazolidinone.

15. 5-Methyl-5-(3-cyclopropylmethoxy-4-methoxyphenyl)-3-isopropyl-2-oxazolidinone.

16. 5-Methyl-5-(3-cyclopropylmethoxy-4-methoxyphenyl)-3-benzyl-2-oxazolidinone.

17. Use of oxazolidone derivatives according to patent Claims 4 to 16 for the preparation of a medicament for the local treatment of inflammations.

**Revendications**

1. Application de pyrrolidones et d'oxazolidinones répondant a la formule générale I:

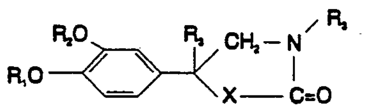

(I),

dans laquelle:

X représente un atome d'oxygène ou un radical méthylène,
$R_1$ et $R_2$ représentent chacun un atome d'hydrogène ou un radical hydrocarboné qui contient au plus 8 atomes de carbone et qui est éventuellement interrompu par un atome d'oxygène,
$R_3$ représente un atome d'hydrogène ou un radical hydrocarboné qui contient au plus 8 atomes de carbone et qui porte éventuellement un radical oxo, et
$R_4$ représente un atome d'hydrogène ou un radical alkyle contenant au plus 4 atomes de carbone, à la fabrication d'un médicament pour le traitement local d'inflammations.

2. Application de pyrrolidones et d'oxazolidinones de formule générale I selon la revendication 1 à la fabrication d'un médicament sous la forme d'une solution, d'une pommade, d'une crème ou d'une lotion pour le traitement local d'inflammations.

3. Application de pyrrolidones et d'oxazolidinones de formule générale I selon la revendication 1 à la fabrication d'un médicament sous la forme d'une poudre pour le traitement local d'inflammations.

4. Oxazolidinones répondant à la formule générale Ia:

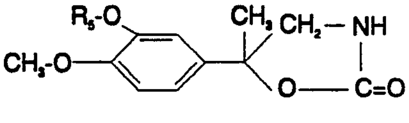

(Ia),

dans laquelle $R_5$ représente un radical hydrocarboné contenant de 2 à 4 atomes de carbone.

5. Méthyl-5 (allyloxy-3 méthoxy-4 phényl)-5-oxazolidinone-2.

6. Méthyl-5 (cyclopropylméthoxy-3 méthoxy-4 phényl)-5 oxazolidinone-2.

7. Méthyl-5 (méthoxy-4 propoxy-3 phényl)-5-oxazolidinone-2.

8. Méthyl-5 (butoxy-3 méthyl-4 phényl)-5-oxazolidinone-2.

9. Méthyl-5 (isopropoxy-3 méthoxy-4 phényl)-5 oxazolidinone-2.

10. Méthyl-5 (éthoxy-2 méthoxy-4 phényl)-5-oxazolidinone-2.

11. Méthyl-5 [méthoxy-4 (tétrahydrofurannyl-3 oxy)-3 phényl]-5 oxazolidinone-2.

12. (Cyclopropylméthoxy-3 méthoxy-4 phényl)-5 diméthyl-3,5 oxazolidinone-2.

13. (Ethoxy-3 méthoxy-4 phényl)-5 diméthyl-3,5 oxazolidinone-2.

14. Méthyl-5 (éthoxy-3 méthoxy-4 phényl)-5-cyclopropylméthyl-3 oxazolidinone-2.

15. Méthyl-5 (cyclopropylméthoxy-3 méthoxy-4 phényl)-5 isopropyl-3 oxazolidinone-2.

16. Méthyl-5 (cyclopropylméthoxy-3 méthoxy-4 phényl)-5 benzyl-3 oxazolidinone-2.

17. Application d'oxazolidinones selon l'une quelconque des revendications 4 à 16 à la fabrication d'un médicament pour le traitement local d'inflammations.